(19) Europäisches Patentamt
European Patent Office
Office européen des brevets

(11) Publication number: 0 049 892
B1

# (12) EUROPEAN PATENT SPECIFICATION

(45) Date of publication of patent specification: 16.01.85

(51) Int. Cl.[4]: C 10 G 3/00, C 07 C 29/15

(21) Application number: 81108247.8

(22) Date of filing: 12.10.81

## (54) Process for the production of gasoline.

(30) Priority: 14.10.80 JP 143266/80

(43) Date of publication of application:
21.04.82 Bulletin 82/16

(45) Publication of the grant of the patent:
16.01.85 Bulletin 85/03

(84) Designated Contracting States:
DE FR IT

(56) References cited:
GB-A-2 016 946
GB-A-2 035 368
US-A-2 628 970
US-A-3 150 942
US-A-4 011 275
US-A-4 048 250
US-A-4 058 576
US-A-4 138 442

(73) Proprietor: TOYO ENGINEERING CORPORATION
2-5, Kasumigaseki 3-chome
Chiyoda-ku, Tokyo 100 (JP)

(72) Inventor: Nozawa, Shinkichi
270-19, Maekaizuka-cho
Funabashi Chiba-ken (JP)
Inventor: Tsujimoto, Masaaki
2-1-2, Midoridai
Funabashi Chiba-ken (JP)

(74) Representative: Schüler, Horst, Dr.
Kaiserstrasse 41
D-6000 Frankfurt/Main 1 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 049 892 B1

## Description

1. Field of the invention

This invention relates to a process for the production of gasoline by synthesizing methanol from a synthesis gas predominantly containing carbon oxides and hydrogen and then synthesizing gasoline from said methanol.

2. Description of the prior art

Process for the production of gasoline via methanol are already known, as disclosed in U.S. Patent Nos. 4,011,275, 4,048,250 and 4,058,576 as well as in UK-patent 2 035 368. According to these processes, methanol and water are condensed and separated from the effluent product of a synthesis reactor, and a part of the residual gas containing carbon oxides, hydrogen and methane is mixed with a freshly fed synthesis gas and recycled to the methanol synthesis reactor. The separated methanol and water are introduced into a gasoline synthesis reactor. Gasoline is condensed and separated from the effluent product of the gasoline synthesis reactor, and the residual gas containing gaseous hydrocarbons is recycled to the gasoline synthesis reactor.

A more simplified and more economical process based on the above-described principle is disclosed in Japanese Patent Laid-Open No. 60588/'80. According to this process, the whole product effluent from a methanol synthesis reactor is fed to a gasoline synthesis reactor. After gasoline boiling range hydrocarbons are condensed and separated from the effluent product of the gasoline synthesis reactor, the greater part of the uncondensed gas consisting largely of carbon oxides and hydrogen and further containing methane and small amounts of $C_2$ to $C_4$ hydrocarbons, together with a freshly fed synthesis gas, is recycled to the methanol synthesis reactor.

Summary of the invention

In order to modify the above-described process in such a way that its methanol synthesis and gasoline synthesis steps can be carried out more efficiently to obtain greater economic advantages, we have performed a series of intensive studies and have perfected the present invention.

Briefly stated, the present invention provides in a process for the production of gasoline by synthesizing methanol from a synthesis gas predominantly containing carbon oxides and hydrogen and then synthesizing gasoline from said methanol, the improvement which comprises feeding the whole product effluent from a methanol synthesis reaction zone to a gasoline synthesis reaction zone; cooling the whole product effluent from said gasoline synthesis reaction zone to condense gasoline and separate it from the uncondensed gas containing carbon oxides, hydrogen and gaseous $C_1$ to $C_4$ hydrocarbons; separating said uncondensed gas into a gas rich in $C_1$ to $C_4$ hydrocarbons and a gas rich in carbon oxides and hydrogen; and recycling said gas rich in carbon oxides and hydrogen, together with a freshly fed synthesis gas, to said methanol synthesis reaction zone.

When the whole product effluent from the gasoline synthesis reaction zone is cooled to condense and separate gasoline, the remaining uncondensed gas still contains trace amounts of aromatic hydrocarbons and $C_5$ or higher aliphatic hydrocarbons which should preferably be removed by adsorption with activated charcoal, silica gel and the like. Thereafter, hollow-fiber separation, molecular sieving, low-temperature separation or the like is used to separate the uncondensed gas into a gas rich in carbon oxides and hydrogen and a gas rich in $C_1$ to $C_4$ hydrocarbons. The gas rich in carbon oxides and hydrogen is mixed with a freshly fed synthesis gas and recycled to the methanol synthesis reaction zone, while the gas rich in lower hydrocarbons is either recycled to the synthesis gas production step or used as fuel.

Thus, the present invention provides a process for the production of gasoline which can achieve higher efficiency and greater economy as compared with prior art processes for synthesizing gasoline via methanol.

Drawing

The single drawing is a flow diagram illustrating one embodiment of the present invention.

Detailed description of the invention

The synthesis gas used as the starting material of methanol synthesis should preferably satisfy the condition

$$(H_2—CO_2)/(CO+CO_2)>2$$

Such synthesis gases can be produced by a number of well-known processes. For example, this may be accomplished by subjecting gaseous or liquid hydrocarbons to catalytic steam reforming. Alternatively, a synthesis gas of desired composition may also be obtained by gasifying said hydrocarbons and/or a solid carbonaceous material, such as coke, coal or the like, through reaction with oxygen and steam, purifying the resulting gas, and subjecting the purified gas to shift reactions, if necessary.

In the process of the present invention, methanol synthesis may be carried out according to any of

several well-known techniques. However, it is preferable to carry out the methanol synthesis at a pressure of 30 to 120 atmospheres and a temperature of 200 to 300°C, as described in Japanese Patent Laid-Open No. 29408/'76. In this case, a well-known catalyst comprising 60 atomic percent of copper, 30 atomic percent of zinc, and 10 atomic percent of aluminum is used.

Gasoline synthesis may likewise be carried out according to any of several well-known techniques. For example, the gasoline synthesis is carried out at a temperature of 250 to 400°C in the zeolite catalyst ZSM-5 as disclosed in U.S. Patent No. 4,048,250. Although this reaction is a highly exothermic one, it is possible to use a reactor as disclosed in Japanese Patent Laid-Open No. 149640/'80 assigned to the same assignee as the present invention.

The present invention will hereinafter be described more fully in connection with the embodiment illustrated in the drawing. However, it is to be understood that the present invention is not limited thereto.

A freshly fed synthesis gas is compressed by means of a compressor 27 and mixed with the gas rich in carbon oxides and hydrogen which gas has been separated in a selective separator 24 and conducted through a pipeline 2. The resulting gas is further compressed by means of a compressor 3 and then heated to a temperature of 200 to 250°C by passing it through a heat exchanger 4. This gas is introduced into a methanol synthesis reactor 7 by way of a pipeline 6. The methanol synthesis reactor 7 is constituted of packed beds arranged in multiple stages. In order to prevent methanol synthesis reactor 7 from being overheated by the heat of the catalytic reaction, a part of the cold gas is conducted through a pipeline 5 branched off before heat exchanger 4 and introduced into methanol synthesis reactor 7 between each stage. All of the gaseous or vaporous product effluent from methanol synthesis reactor 7 is conducted through a pipeline 8, passed through a heat exchanger 9 to preheat it to a temperature of 250 to 450°C, and then introduced into a gasoline synthesis reactor 11. The gasoline synthesis reaction is carried out in the presence of a well-known zeolite catalyst. Since this reaction is a highly exothermic one, indirect cooling is required to remove surplus heat of reaction. In this embodiment, the surplus heat of reaction is removed by passing a mixture of steam and hot water through a radial flow reactor. More specifically, a steam drum 30 is provided with boiler water which has been purified by thorough chemical processing. The water within steam drum 30 is conducted through a pipeline 13, pressurized by means of a circulating pump 29, and fed through a pipeline 14 to heat transfer tubes incorporated in gasoline synthesis reactor 11. By the action of the heat of reaction, the water flowing through the heat transfer tubes changes into a mixture of steam and hot water, which is conducted through a pipeline 15 and returned to steam drum 30. In steam drum 30, steam is separated and discharged from the system by way of a pipeline 16, while the remaining hot water is again circulated through the system with the aid of circulating pump 29. Water is continuously supplied so as to maintain a constant water level within steam drum 30. The steam discharged from the system via pipeline 16 is saturated steam having a pressure of 25 to 150 atmospheres.

The gaseous or vaporous product effluent from gasoline synthesis reactor 11 is conducted through a pipeline 12 and then cooled by passing it successively through heat exchangers 9 and 4. Thereafter, the product is further cooled in a cooler 19 and then introduced into a separator 21, where gasoline boiling range hydrocarbons are separated in the form of a condensate and withdrawn from the system by way of a pipeline 22. Since the remaining uncondensed gas still contains trace amounts of aromatic hydrocarbons and $C_5$ or higher aliphatic hydrocarbons, they are removed by a suitable means (not shown) for adsorbing them with activated charcoal, silica gel and the like. Then, the uncondensed gas is introduced into a selective separator 24 which, as stated before, may be based on any of several well-known separation techniques such as hollow-fiber separation molecular sieving, low-temperature separation and the like. In this selective separator 24, the uncondensed gas is separated into a gas rich in carbon oxides and hydrogen and a gas rich in lower, or $C_1$ to $C_4$, hydrocarbons. The former is conducted through pipeline 2 and recycled to methanol synthesis reactor 7, while the latter is either returned through a pipeline 25 to the equipment for the production of a synthesis gas or used as fuel.

If lower, or $C_1$ to $C_4$, hydrocarbons are formed during the gasoline synthesis reaction, the degree of conversion of methanol to gasoline will be lowered. The conversion of methanol to lower hydrocarbons can be suppressed by previously adding a gas rich in lower hydrocarbons to the feed gas of gasoline synthesis reactor 11. Moreover, the conversion of methanol to hydrocarbons can be expressed by the reaction formulas

$$2CH_3OH \rightarrow CH_3OCH_3 + H_2O$$

$$CH_3OCH_3 \rightarrow -CH_2 \cdot CH_2- + H_2O$$

Thus, this is a consecutive reaction in which methanol is converted to higher gasoline boiling range hydrocarbons via lower hydrocarbons. Accordingly, it is preferable to add lower, or $C_1$ to $C_4$, hydrocarbons to gasoline synthesis reactor 11 for the purpose of suppressing the conversion of methanol to lower hydrocarbons and promoting the consecutive reaction for converting lower hydrocarbons to higher ones. In the process of the present invention, this can readily be accomplished by withdrawing the gas rich in lower hydrocarbons from selective separator 24 and conducting it

through a pipeline 31 into gasoline synthesis reactor 11. In this manner, the conversion of methanol to lower hydrocarbons can be prevented and the yield of gasoline can be increased. Moreover, since the inert gas content of the feed gas used in the methanol synthesis step is decreased, the reaction conditions for methanol synthesis can be modified so as to obtain greater economic advantages.

The present invention is further illustrated by the following example.

Example

Using the equipment illustrated in the drawing, 32.27 $Nm^3/hr$ of a synthesis gas obtained by steam reforming of natural gas and having the composition shown in Table 1 was mixed with 64.23 $Nm^3/hr$ of a gas rich in carbon oxides and hydrogen which gas was withdrawn from selective separator 24 and had the composition shown in Table 7. Thus, a mixed gas having the composition shown in Table 2 was obtained.

TABLE 1

| | $CO_2$ | CO | $H_2$ | $CH_4$ |
|---|---|---|---|---|
| mole % | 5.67 | 15.53 | 74.93 | 3.87 |

TABLE 2

| | $CO_2$ | CO | $H_2$ | $CH_4$ |
|---|---|---|---|---|
| mole % | 3.94 | 5.70 | 88.81 | 1.55 |

This mixed gas was compressed to 60 atmospheres, heated to 240°C, and then introduced into methanol synthesis reactor 7. The methanol synthesis was carried out in the presence of 8 liters of a catalyst comprising 60 atomic percent of copper, 30 atomic percent of zinc, and 10 atomic percent of aluminum. As a result, 88.5 $Nm^3/hr$ of a gas having the composition shown in Table 3 was withdrawn from the outlet of the methanol synthesis reactor 7.

TABLE 3

| | $CO_2$ | CO | $H_2$ | $CH_4$ | $CH_3OH$ | $H_2O$ |
|---|---|---|---|---|---|---|
| mole % | 3.39 | 2.60 | 86.89 | 1.69 | 4.52 | 0.90 |

The aforesaid gas was further heated to 375°C and then introduced into gasoline synthesis reactor 11 packed with the zeolite catalyst ZSM-5 as described in U.S. Patent No. 4,048,250. Although this reaction was a highly exothermic one, the heat transfer tubes disposed within the catalyst chamber served to prevent the catalyst from being overheated and keep it at appropriate temperatures. Within the heat transfer tubes was produced a mixture of steam and hot water, which was introduced into steam drum 30 and separated to yield 40 kg/hr of steam having a pressure of 100 $kg/cm^2$.

The whole reaction product effluent from gasoline synthesis reactor 11 was cooled to room temperature to yield 83.85 $Nm^3/hr$ of uncondensed gas having the composition shown in Table 4 and 6.07 kg/hr of condensate having the composition shown in Table 5.

TABLE 4

| | $CO_2$ | CO | $H_2$ | $CH_4$ | $C_2H_6$ | $C_3H_8$ | $C_4H_{10}$ |
|---|---|---|---|---|---|---|---|
| Uncondensed gas, mole % | 3.58 | 2.74 | 91.71 | 1.79 | 0.04 | 0.07 | 0.07 |

TABLE 5

| | $H_2O$ | $C_4H_{10}$ | $C_5$ and higher hydrocarbons | Aromatics |
|---|---|---|---|---|
| Condensate, wt. % | 63.59 | 1.53 | 25.44 | 9.44 |

The gasoline boiling range hydrocarbon fraction of this condensate had the composition shown in Table 6.

TABLE 6

| | Paraffins | Naphthenes | Olefins | Aromatics |
|---|---|---|---|---|
| wt. % | 53 | 12 | 7 | 28 |

Since the uncondensed gas still contained trace amounts of $C_5$ or higher aliphatic hydrocarbons and aromatic hydrocarbons, they were removed by adsorption with silica gel, activated charcoal and the like. Thereafter, the uncondensed gas was introduced into selective separator 24. The selective separator used in this example was of the hollow-fiber type. Such gases as $H_2$, CO, $CO_2$ and the like easily diffused through the walls of the hollow fibers and accumulated within the bores thereof, thus yielding 64.23 $Nm^3/hr$ of a gas rich in carbon oxides and hydrogen. On the other hand, $C_1$ to $C_4$ hydrocarbons hardly diffused through the walls of the hollow fibers, thus yielding 19.62 $Nm^3/hr$ of a gas enriched with these hydrocarbons. The compositions of these gases are shown in Table 7.

TABLE 7

| | $CO_2$ | CO | $H_2$ | $CH_4$ | $C_2H_6$ | $C_3H_8$ | $C_4H_{10}$ |
|---|---|---|---|---|---|---|---|
| Gas rich in carbon oxides and hydrogen, mole% | 3.07 | 0.76 | 95.78 | 0.39 | | | |
| Gas rich in hydrocarbons, mole % | 5.25 | 9.23 | 78.40 | 6.37 | 0.16 | 0.29 | 0.30 |

The gas rich in carbon oxides and hydrogen was mixed with a freshly fed synthesis gas and recycled to methanol synthesis reactor 7, while the gas rich in hydrocarbons was returned to the reformer used for the production of a synthesis gas.

**Claims**

1. Process for the production of gasoline by synthesizing methanol from a synthesis gas predominantly containing carbon oxides and hydrogen, feeding the whole product effluent from a methanol synthesis reaction zone to a gasoline synthesis reaction zone, cooling the whole product from said gasoline synthesis reaction zone to condense and separate gasoline, and recycling again the uncondensed gas to a methanol synthesis reaction zone, characterized in that there is selectively separated said uncondensed gas into a gas rich in $C_1$ to $C_4$ hydrocarbons and a gas rich in carbon oxides and hydrogen; and recycling said gas rich in carbon oxides and hydrogen, together with a freshly fed synthesis gas, to said methanol synthesis reaction zone.

2. Process as claimed in Claim 1 wherein, after the separation of gasoline, a hollow-fiber separator is used to separate said uncondensed gas into a gas rich in $C_1$ to $C_4$ hydrocarbons and a gas rich in carbon oxides and hydrogen.

3. Process as claimed in Claim 1 wherein, after the separation of gasoline, a molecular sieve is used to separate said uncondensed gas into a gas rich in $C_1$ to $C_4$ hydrocarbons and a gas rich in carbon oxides and hydrogen.

**Patentansprüche**

1. Verfahren zur Herstellung von Benzin durch Synthetisieren von Methanol aus einem Synthesegas, das vorwiegend Kohlenstoffoxide und Wasserstoff enthält, Einführen des gesamten Produktauslaufs aus einer Methanolsynthesereaktionszone in eine Benzinsynthesereaktionszone, Abkühlen des gesamten Produktes von dieser Benzinsynthesereaktionszone, um Benzin zu kondensieren und abzutrennen, und wieder Rückführen des nichtkondensierten Gases zu einer Methanolsynthesereaktionszone, dadurch gekennzeichnet, daß das nichtkondensierte Gas selektiv in ein Gas, das reich an $C_1$ bis $C_4$ Kohlenwasserstoffen ist, und ein Gas, das reich an Kohlenstoffoxiden und Wasserstoff ist, getrennt wird, und das Gas, das reich an Kohlenstoffoxiden und Wasserstoff ist, zusammen mit einem frisch zugeführten Beschickungssynthesegas zu der Methanolsynthesereaktionszone zurückgeführt wird.

2. Verfahren nach Anspruch 1, dadurch gekennzeichnet, daß nach der Abtrennung von Benzin eine Hohlfasertrennanlage verwendet wird, um das nichtkondensierte Gas in ein Gas, das reich an $C_1$ bis $C_4$ Kohlenwasserstoffen ist, und ein Gas, das reich an Kohlenstoffoxiden und Wasserstoff ist, zu trennen.

3. Verfahren nach Anspruch 1, dadurch gekennzeichnet, daß nach der Abtrennung von Benzin ein Molekularsieb verwendet wird, um das nichtkondensiert Gas in ein Gas, das reich an $C_1$ bis $C_4$ Kohlenwasserstoffen ist, und ein Gas, das reich an Kohlenstoffoxiden und Wasserstoff ist, zu trennen.

**Revendications**

1. Procédé de production d'essence par syntèse du méthanol à partir d'un gaz de synthèse contenant principalement des oxydes de carbone et de l'hydrogène, introduction de tout l'effluent de produit provenant d'un zone de réaction de synthèse de méthanol dans une zone de réaction de

synthèse d'essence, refroidissement de tout le produit de la zone de réaction de synthèse d'essence pour condenser et séparer l'essence et recyclage du gaz non condensé vers la zone de réaction de synthèse du méthanol, caractérisé en ce que l'on sépare sélectivement ce gaz non condensé en un gaz riche en hydrocarbures en $C_1$ à $C_4$ et en un gaz riche en oxydes de carbone et en hydrogène; et que l'on recycle ce gaz riche en oxydes de carbone et en hydrogène, avec un gaz de synthèse fraîchement introduit, vers la zone de réaction de synthèse du méthanol.

2. Procédé suivant la revendication 1, caractérisé en ce qu'après avoir sépare l'essence, on utilise un séparateur à fibre creuse pour séparer le gaz non condensé en un gaz riche en hydrocarbures en $C_1$ à $C_4$.

3. Procédé suivant la revendication 1, caractérisé en ce qu'après avoir séparé l'essence, on utilise un tamis moléculaire pour séparer le gaz non condensé en un gaz riche en hydrocarbures en $C_1$ à $C_4$ et en un gaz riche en oxydes de carbone et en hydrogène.

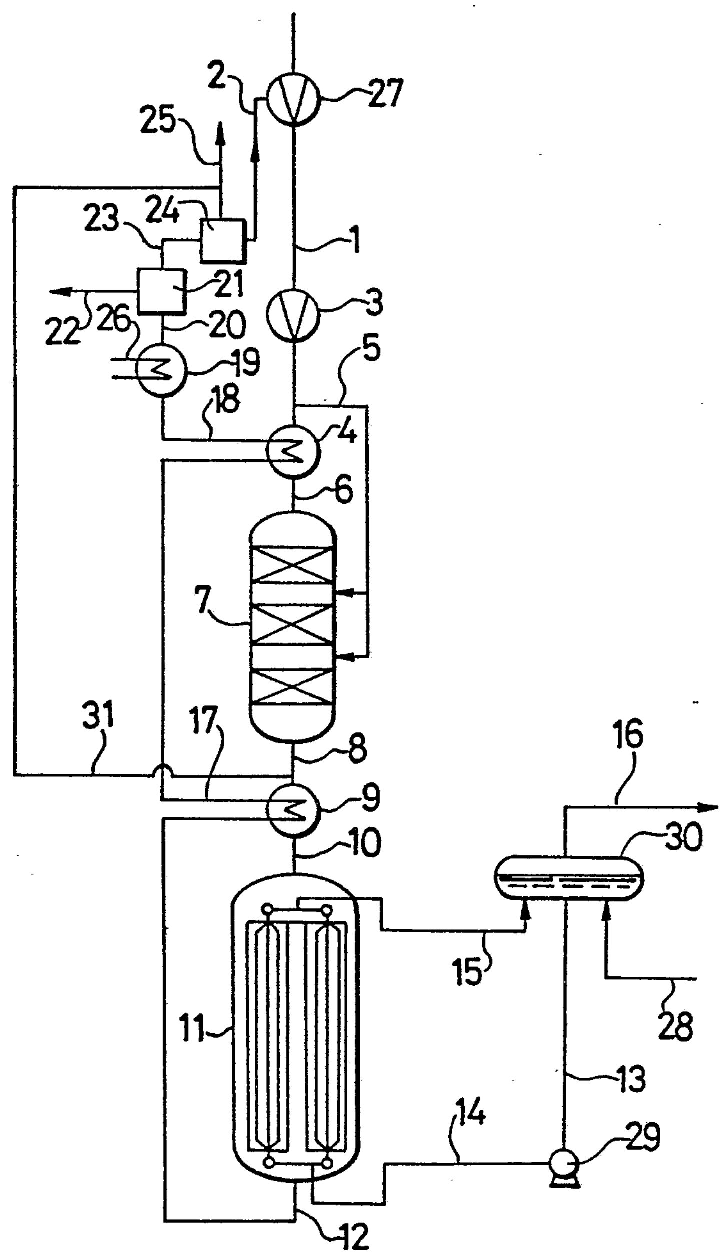
2
27
25
24
23
1
21
3
22
26
20
19
5
18
4
6
7
16
31
17
8
9
30
10
15
11
28
13
14
29
12